# EUROPEAN PATENT SPECIFICATION

(11) **EP 4 062 863 B1**
(45) Date of publication and mention of the grant of the patent: **04.03.2026**
(21) Application number: 20891225.3
(22) Date of filing: 18.11.2020
(51) Int. Cl.: A61C 7/08

(54) **ORTHODONTIC ALIGNER AND MANUFACTURING METHOD THEREOF**
ORTHODONTISCHE SCHIENE UND VERFAHREN ZU IHRER HERSTELLUNG
ALIGNEUR DENTAIRE ET PROCÉDÉ DE FABRICATION DE CELUI-CI

(30) Priority: 18.11.2019 JP 2019208222
(43) Date of publication of application: 28.09.2022
(73) Proprietor: Kuraray Noritake Dental Inc., Kurashiki-shi, Okayama 710-0801 (JP)
(72) Inventor: ITO Misaki, Tainai-shi, Niigata 959-2653 (JP); SUZUKI Kenji, Tainai-shi, Niigata 959-2653 (JP)
(74) Representative: D Young & Co LLP
(86) International application number: PCT/JP2020/042974
(87) International publication number: WO 2021/100752

(56) References cited:
- WO-A1-2018/118200
- CN-U- 206 194 240
- JP-A- 2017 046 793
- JP-A- H08 196 551
- US-A1- 2018 126 626
- US-B2- 8 152 518

## Description

### TECHNICAL FIELD

The present disclosure relates to a dental intraoral device to be worn within an oral cavity, and a method for manufacturing the dental intraoral device.

### BACKGROUND ART

Conventionally, a dental intraoral device having holes in tooth portions has been known (e.g., see Patent Literature 1).

Patent Literature 1 discloses a dental device configured to substantially expose an occlusal surface of the dentition of a patient when worn by the patient. This can improve hygiene due to the inflow of saliva and air between teeth. Patent Literature 2 discloses an ideal occlusal structure function recovery device comprising a metal mesh.

### CITATION LIST

### Patent Literature

Patent Literature 1: JP 2018-504191 A
Patent Literature 2: JP H08 196551 A

Relevant prior art is also exemplified by US 8 152 518 B2 and US 2018/126626 A1.

### SUMMARY

### Technical Problem

However, the problem with the dental device according to Patent Literature 1 is that large holes are formed to expose the occlusal surfaces, and the required strength cannot be ensured. If a resin covering a tooth neck (cervix) at a facial (buccal) side and a resin covering a tooth neck (cervix) at a lingual side or a palatal side cannot be coupled together at the occlusal surface, it is difficult for tensile force to be produced between the resins, impairing strength.

Accordingly, an object of the present disclosure is to provide a dental intraoral device that can ensure the required strength even with improved hygiene.

### Solution to Problem

To achieve the above object, a dental intraoral device of the present disclosure is a dental intraoral device to be worn within an oral cavity and includes a resin tooth portion, wherein at least a part of the tooth portion is formed into a mesh shape.

The present invention is set out in the appended claims.

### Advantageous Effects

The dental intraoral device of the present disclosure having such a configuration can ensure a required strength even with improved hygiene.

### BRIEF DESCRIPTION OF DRAWINGS

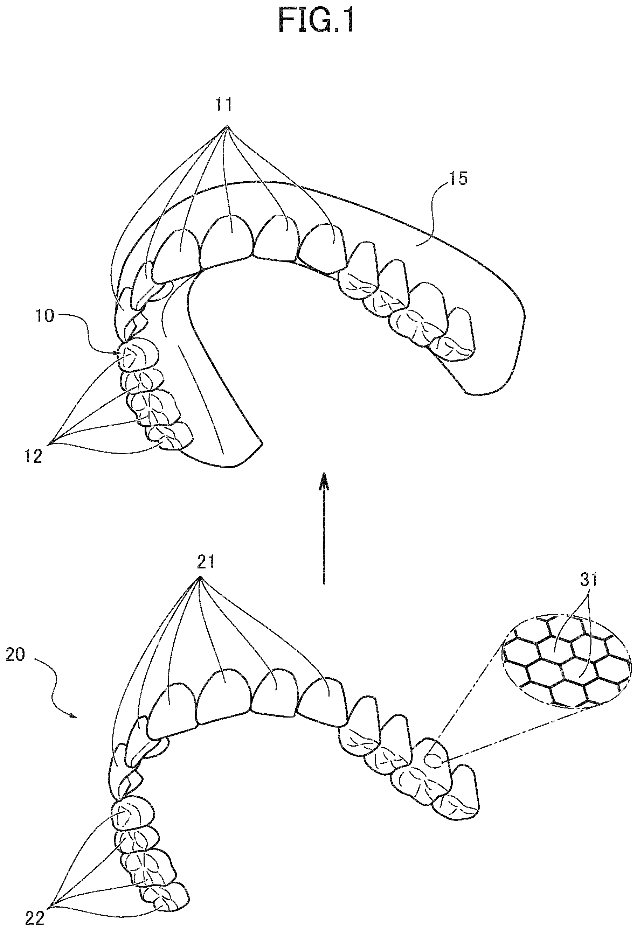

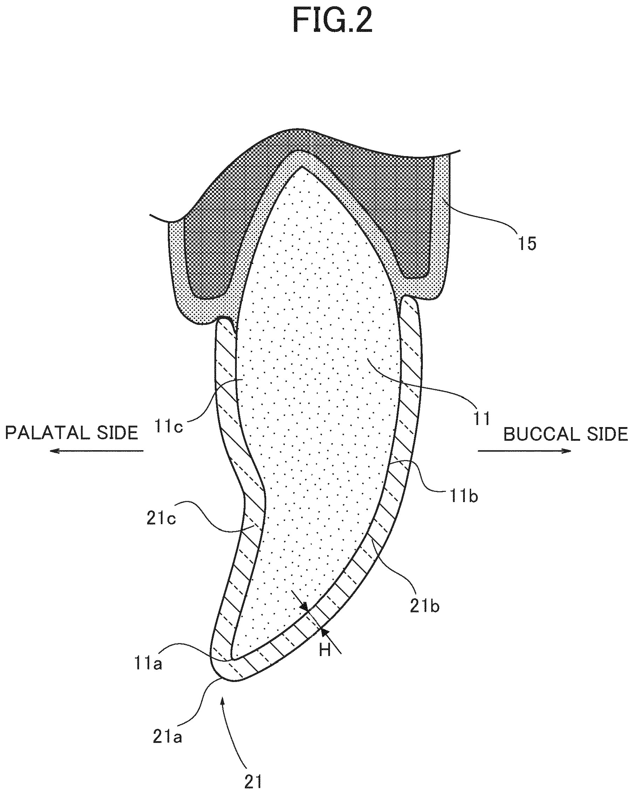

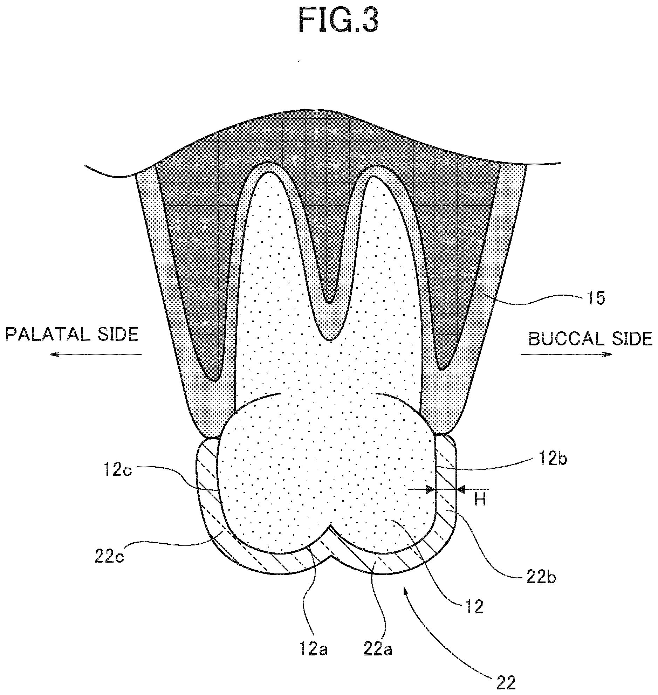

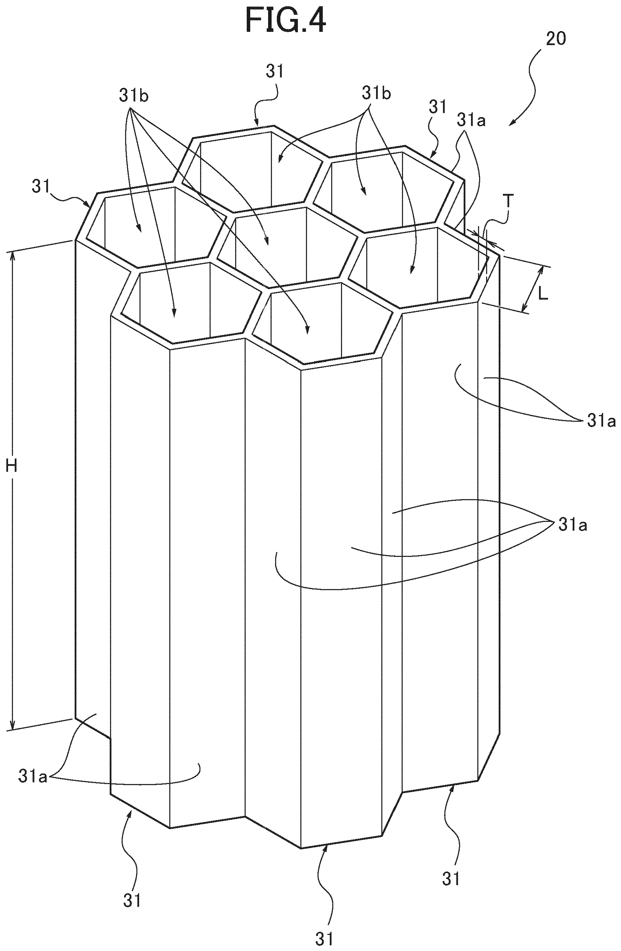

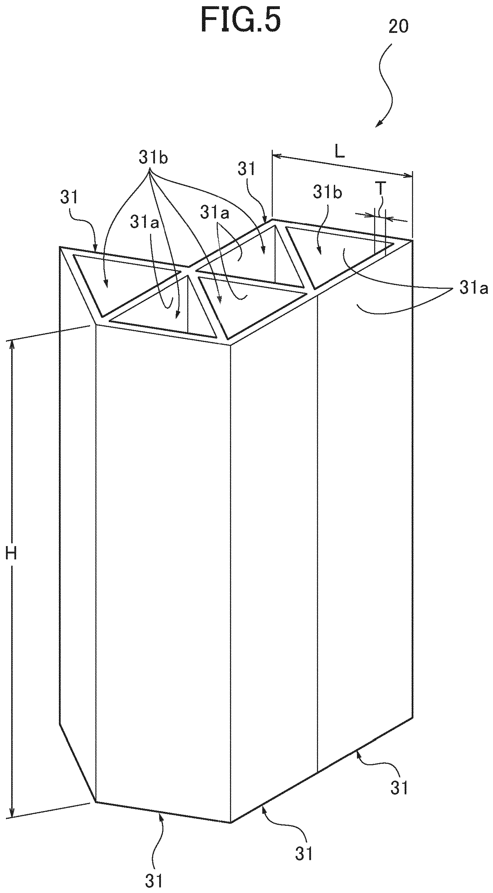

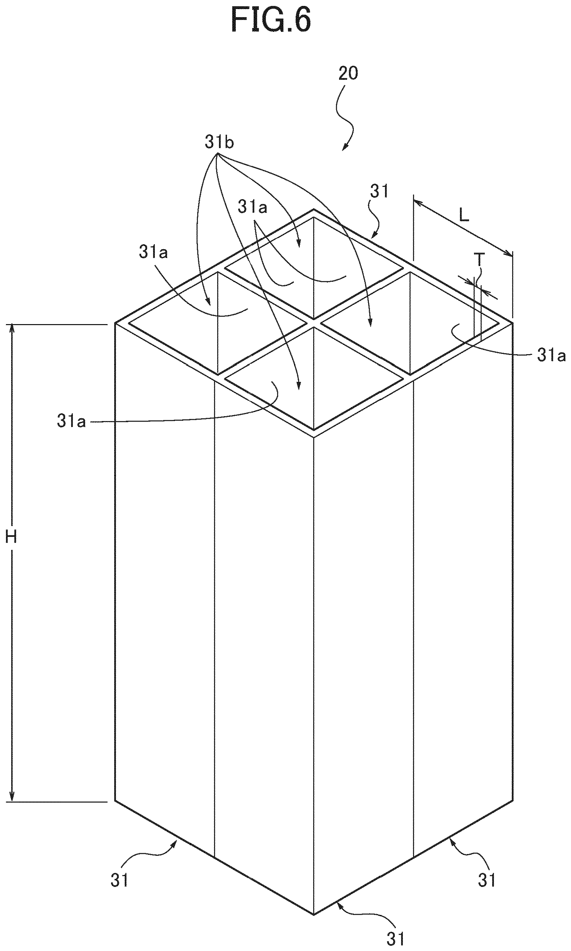

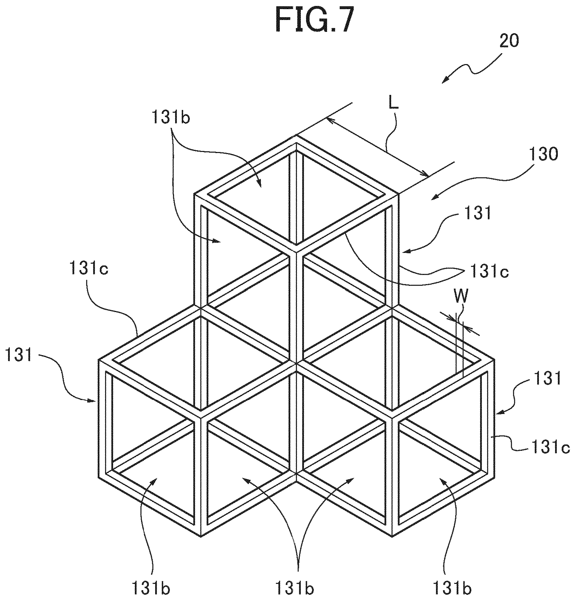

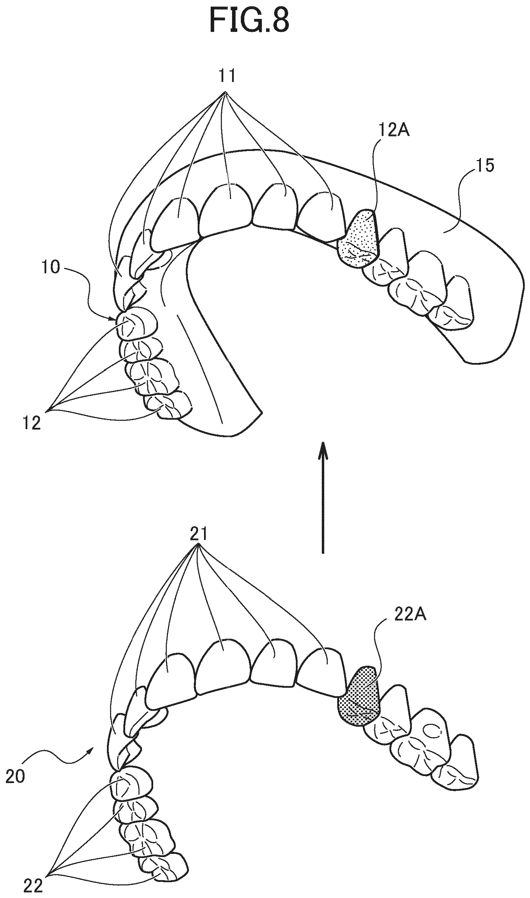

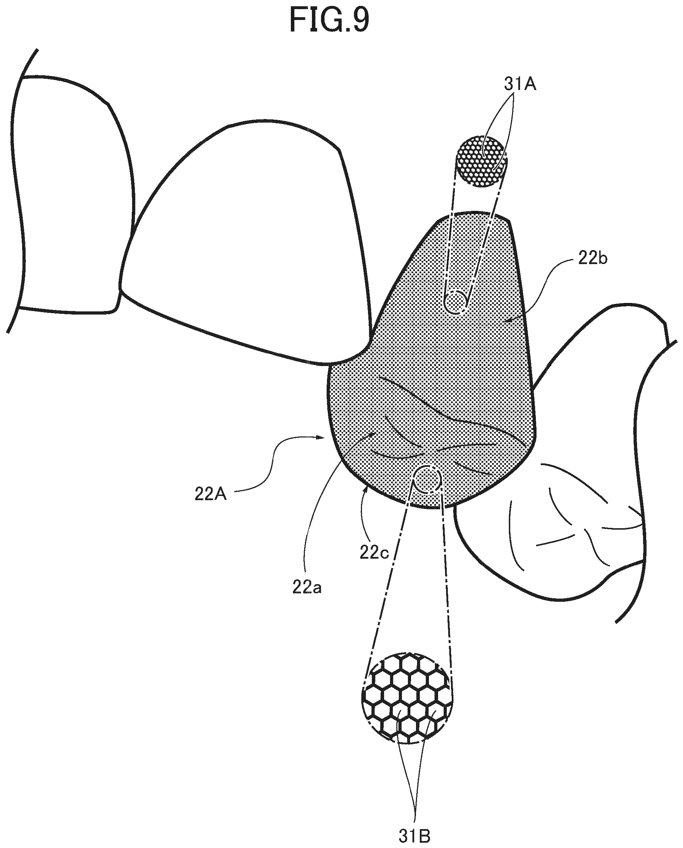

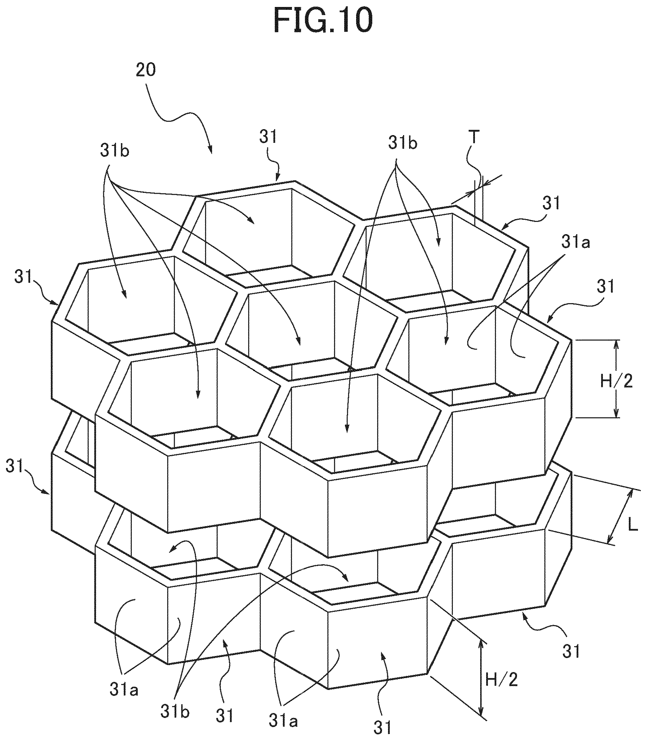

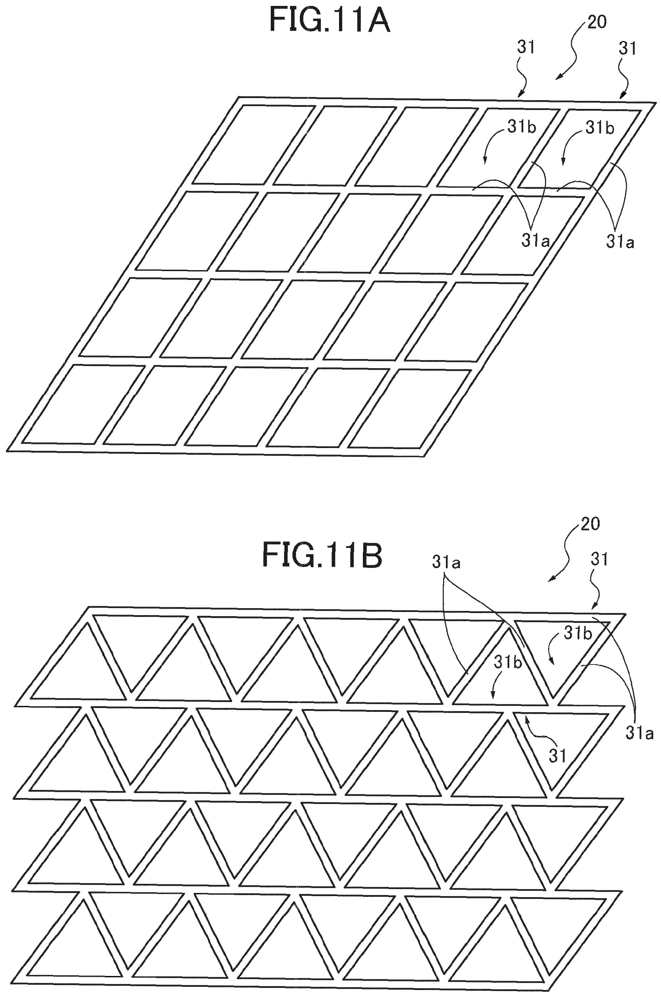

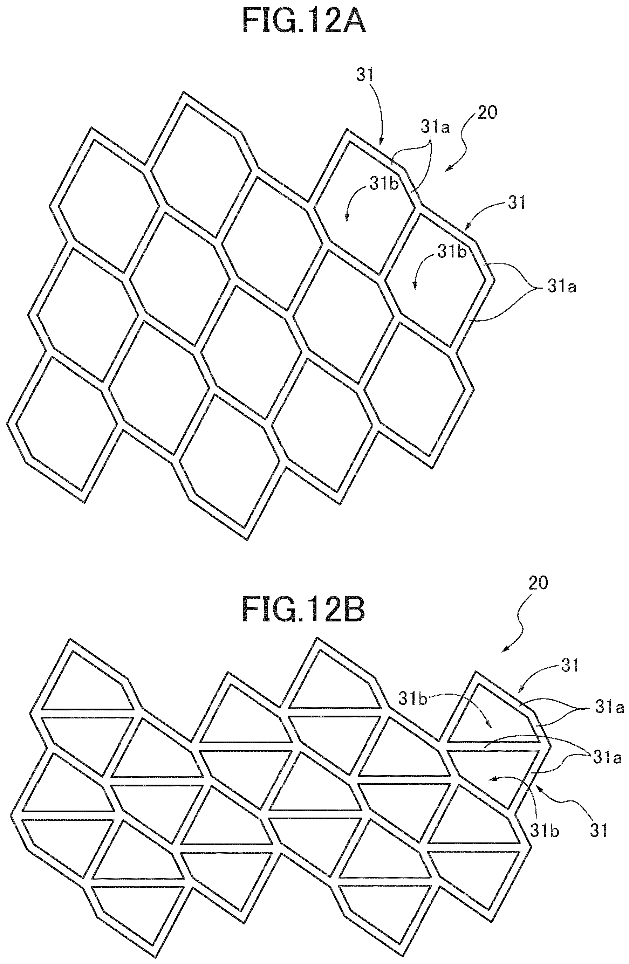

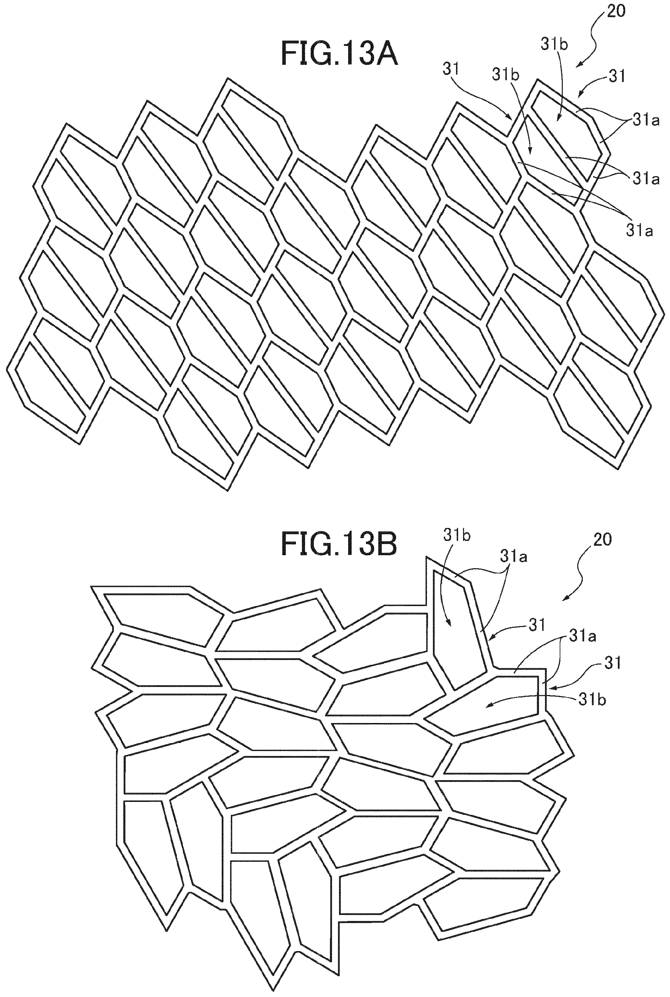

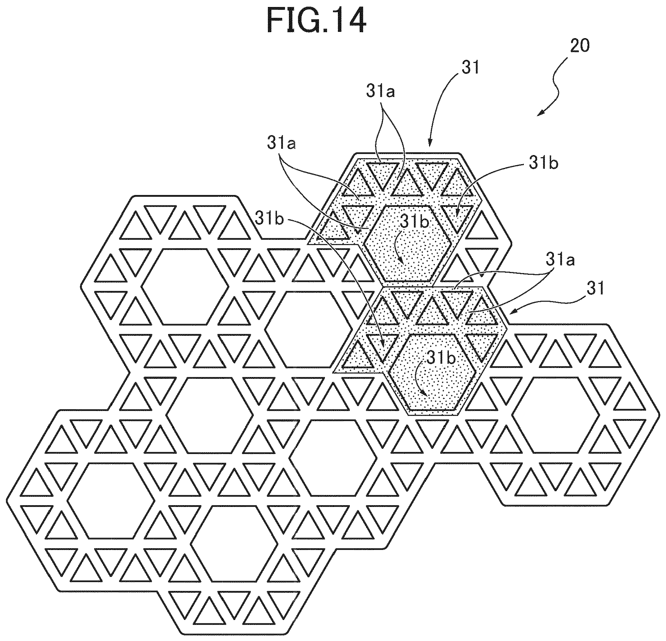

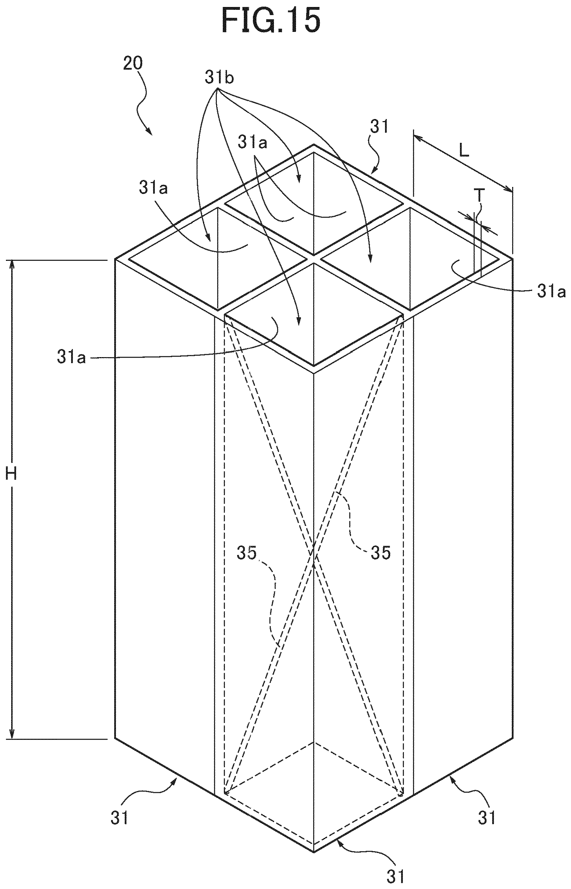

FIG. 1 is an exploded perspective view illustrating an orthodontic aligner according to a first embodiment and an upper jaw. FIG. 2 is a cross-sectional view of an anterior tooth illustrating a state where the orthodontic aligner according to the first embodiment is worn within an oral cavity. FIG. 3 is a cross-sectional view of a molar tooth illustrating a state where the orthodontic aligner according to the first embodiment is worn within the oral cavity. FIG. 4 is a perspective view illustrating unit regions of a tooth portion according to the first embodiment. FIG. 5 is a perspective view illustrating unit regions according to a modified example of the first embodiment. FIG. 6 is a perspective view illustrating unit regions according to another modified example of the first embodiment. FIG. 7 is a perspective view illustrating unit regions of a tooth portion according to a second embodiment. FIG. 8 is an exploded perspective view illustrating an orthodontic aligner according to a third embodiment and the upper jaw. FIG. 9 is a perspective view illustrating a tooth portion covering a tooth to be straightened according to the third embodiment. FIG. 10 is a perspective view illustrating unit regions according to another example. FIG. 11A is a plan view illustrating unit regions according to another example. FIG. 11B is a plan view illustrating unit regions according to another example. FIG. 12A is a plan view illustrating unit regions according to another example. FIG. 12B is a plan view illustrating unit regions according to another example. FIG. 13A is a plan view illustrating unit regions according to another example. FIG. 13B is a plan view illustrating unit regions according to another example. FIG. 14 is a plan view illustrating unit regions according to another example. FIG. 15 is a perspective view illustrating unit regions according to another example.

### DESCRIPTION OF EMBODIMENT

Hereinafter, embodiments of a dental intraoral device according to the present disclosure are described with reference to first to third embodiments illustrated in the drawings.

### First Embodiment

A dental intraoral device in the first embodiment is applied to an orthodontic aligner to be worn within an oral cavity to cover upper teeth.

(Configuration of Orthodontic Aligner) FIG. 1 is an exploded perspective view illustrating an orthodontic aligner according to the first embodiment and an upper jaw. FIG. 2 is a cross-sectional view of an anterior tooth illustrating a state where the orthodontic aligner according to the first embodiment is worn within an oral cavity. FIG. 3 is a cross-sectional view of a molar tooth illustrating a state where the orthodontic aligner according to the first embodiment is worn within the oral cavity. FIG. 4 is a perspective view illustrating unit regions of a tooth portion according to the first embodiment. FIG. 5 is a perspective view illustrating unit regions according to a modified example of the first embodiment. FIG. 6 is a perspective view illustrating unit regions according to another modified example of the first embodiment. The configuration of the orthodontic aligner according to the first embodiment is described below.

An orthodontic aligner 20 is formed by a three-dimensional modeling device based on the three-dimensional data. The orthodontic aligner 20 is worn on teeth 10 before straightening, and the teeth 10 are straightened to a desired straightening position.

As illustrated in the upper drawing in FIG. 1, the teeth 10 are supported by a gum 15 embracing the roots of the teeth 10. The teeth 10 include front teeth or anterior teeth 11 and molar teeth 12.

The orthodontic aligner 20 includes an anterior tooth portion 21 for covering the anterior teeth 11, and a molar tooth portion 22 for covering the molar teeth 12.

As illustrated in the lower drawing in FIG. 1 and FIG. 2, the anterior tooth portion 21 includes a tip portion 21a, a buccal-side (facial-side) portion 21b, and a palatal-side portion 21c and is formed into a concave shape (concave groove shape) with these portions. The tip portion 21a is a top portion that covers a tip 11a of the anterior tooth 11. The buccal-side portion 21b is a side portion that covers a buccal-side (facial-side) surface 11b of the anterior tooth 11. The palatal-side portion 21c is a side portion that covers a palatal-side surface 11c of the anterior tooth 11.

As illustrated in FIG. 3, the molar tooth portion 22 includes an occlusal portion 22a, a buccal-side (facial-side) portion 22b, a palatal-side portion 22c, and is formed into a concave shape with these portions. The occlusal portion 22a is a top portion that covers an occlusal surface 12a of the molar tooth 12. The buccal-side portion 22b is a side portion that covers a buccal-side (facial-side) surface 12b of the molar tooth 12. The palatal-side portion 22c is a side portion that covers a palatal-side surface 12c of the molar tooth 12.

As illustrated in FIGS. 1 and 4, the anterior tooth portion 21 and the molar tooth portion 22 are each formed into a mesh shape by approximately regularly arranging the unit regions 31 each of which includes a hole 31b. The mesh shape means a regular or at least partially random three-dimensional mesh structure. Note that FIG. 1 partially illustrates the unit regions 31 in an enlarged view. The anterior tooth portion 21 and the molar tooth portion 22 are entirely formed into a mesh shape in the first embodiment. However, the anterior tooth portion 21 and the molar tooth portion 22 may be partially formed into a mesh shape. FIGS. 4 to 6 illustrate examples of the unit regions 31.

(Regular Hexagon Unit Region) As illustrated in FIG. 4, the cross-sectional shape of each of the unit regions 31 is formed by a regular hexagon in the present embodiment. The regular hexagon is a regular polygon that can tessellate (realize a tessellation) with one type of the polygon (polygonal shape) In the top portion, each of the unit regions 31 is formed in a tubular or cylindrical shape by side walls 31a, which define a regular hexagon in a plan view, and includes the hole 31b that extends in a vertical direction. In the side portions, each of the unit regions 31 is formed in a tubular or cylindrical shape by the side walls 31a, which define a regular hexagon in a side view, and includes the hole 31b that extends a horizontal direction.

In the anterior tooth portion 21, the tessellation is done by the unit regions 31 along the tip 11a, the buccal-side surface 11b, and the palatal-side surface 11c of the anterior tooth 11. Moreover, in the molar tooth portion 22, the tessellation is done by the unit regions 31 along the occlusal surface 12a, the buccal-side surface 12b, and the palatal-side surface 12c of the molar tooth 12.

A length L of each side of the unit region 31 is preferably, for example, 0.1 [mm] or more in terms of the passability of saliva, and is preferably, for example, 5.0 [mm] or less in terms of the securing of its strength. A height H of the unit region 31 may be set to a predetermined value (e.g., 0.05 [mm]). A thickness T of the side wall 31a may be set to a predetermined value (e.g., 0.02 [mm]). A ratio L/T of the thickness T to the length L is preferably 1.25 to 50, more preferably 1.5 to 40, and yet more preferably 2.0 to 30. The saliva easily passes when the ratio L/T of the thickness T to the length L is 1.25 or more. The side walls 31a that define the sides become difficult to buckle when the ratio L/T of the thickness T to the length L is 50 or less.

(Regular Triangle Unit Region) As illustrated in FIG. 5, the cross-sectional shape of each of the unit regions 31 in another example may be formed by a regular triangle. The regular triangle is a regular polygon that can tessellate (realize the tessellation) with one type of the polygon (polygonal shape). In the top portion, each of the unit regions 31 is formed in the tubular or cylindrical shape by the side walls 31a, which define a regular triangle in a plan view, and includes the hole 31b that extends in the vertical direction. In the side portion, each of the unit regions 31 is formed in the tubular or cylindrical shape by the side walls 31a, which define the regular triangle in a side view, and includes the hole 31b that extends the horizontal direction.

In the anterior tooth portion 21, the tessellation is done by the unit regions 31 along the tip 11a, the buccal-side surface 11b, and the palatal-side surface 11c of the anterior tooth 11. Moreover, in the molar tooth portion 22, the tessellation is done by the unit regions 31 along the occlusal surface 12a, the buccal-side surface 12b, and the palatal-side surface 12c of the molar tooth 12.

The length L of each side of the unit region 31 is preferably, for example, 0.1 [mm] or more in terms of the passability of saliva, and preferably 5.0 [mm] or less in terms of the securing of its strength. The height H of the unit region 31 may be set to a predetermined value (e.g., 0.05 [mm]). The thickness T of the side wall 31a may be set to a predetermined value (e.g., 0.02 [mm]). The ratio L/T of the thickness T to the length L is preferably 2.5 to 100, more preferably 3.0 to 90, and yet more preferably 3.5 to 80. The saliva easily passes when the ratio L/T of the thickness T to the length L is 2.5 or more. The side walls 31a that define the sides becomes difficult to buckle when the ratio L/T of the thickness T to the length L is 100 or less.

(Regular Tetragon Unit Region) As illustrated in FIG. 6, the cross-sectional shape of each of the unit regions 31 in another different example may be formed by a regular tetragon. The regular tetragon is a regular polygon that can tessellate (realize a tessellation) with one type of the polygon. In the top portion, each of the unit regions 31 is formed in the tubular or cylindrical shape by the side walls 31a, which define a regular tetragon in a plan view, and includes the hole 31b that extends in the vertical direction. In the side portion, each of the unit regions 31 is formed in the tubular or cylindrical shape by the side walls 31a, which define the regular tetragon in a side view, and includes the hole 31b that extends the horizontal direction.

In the anterior tooth portion 21, the tessellation is done by the unit regions 31 along the tip 11a, the buccal-side surface 11b, and the palatal-side surface 11c of the anterior tooth 11. Moreover, in the molar tooth portion 22, the tessellation is done by the unit regions 31 along the occlusal surface 12a, the buccal-side surface 12b, and the palatal-side surface 12c of the molar tooth 12.

The length L of each side of the unit region 31 is preferably, for example, 0.1 [mm] or more in terms of the passability of saliva, and preferably 5.0 [mm] or less in terms of the securing of strength. The height H of the unit region 31 may be set to a predetermined value (e.g., 0.05 [mm]). The thickness T of the side wall 31a may be set to a predetermined value (e.g., 0.02 [mm]). The ratio L/T of the thickness T to the length L is preferably 2.0 to 80, more preferably 2.5 to 70, and yet more preferably 3.0 to 60. The saliva easily passes when the ratio L/T of the thickness T to the length L is 2.0 or more. The side walls 31a that define the sides become difficult to buckle when the ratio L/T of the thickness T to the length L is 80 or less.

The orthodontic aligner 20 is additively manufactured by applying ultraviolet laser light to a photo-curing resin by using the three-dimensional modeling device based on three-dimensional data of the orthodontic aligner 20 previously generated by three-dimensional software. For example, a resin containing a radical polymerizable compound such as a meth(acrylic) monomer, a polymerizable monomer including a cationic polymerization compound such as an epoxy compound, and a photopolymerization initiator may be used as the photo-curing resin.

The orthodontic aligner 20 as configured above is worn to cover the upper teeth 10. The teeth 10 wearing the orthodontic aligner 20 are straightened to a desired straightening position.

A plurality of the orthodontic aligners 20 is prepared to straighten the teeth 10 to the final desired straightening position step by step.

Note that the cross-sectional shape of the unit region 31 is not limited to a regular hexagon, a regular triangle, or a regular tetragon, and may be a regular polygon that can tessellate (realize the tessellation) with one type of the polygon (polygonal shape).

(Effect of Dental Intraoral Device) The effect of the dental intraoral device (orthodontic aligner 20) according to the first embodiment is described below. The dental intraoral device (orthodontic aligner 20) according to the first embodiment is a dental intraoral device (orthodontic aligner 20) to be worn within the oral cavity and includes a resin tooth portion (anterior tooth portion 21, molar tooth portion 22). At least a part of the tooth portion (anterior tooth portion 21, molar tooth portion 22) is formed into a mesh shape (FIG. 1).

By forming the tooth portion (anterior tooth portion 21, molar tooth portion 22) into the mesh shape, the occlusal surface can be exposed while a predetermined strength is ensured. Thus, hygiene can be improved by the inflow of saliva and interdental air. Moreover, the feeling of a foreign object when wearing the dental intraoral device (orthodontic aligner 20) can be reduced, which improves wearability and reduces the feeling of nausea and vomiting.

Moreover, it is generally important in an orthodontic aligner that a resin covering a tooth neck (cervix) at a buccal (facial) side and a resin covering a tooth neck (cervix) at the lingual side or palatal side are coupled together to properly exert straightening or orthodontic force. When one of the resin covering the tooth neck (cervix) at the buccal (facial) side and the resin covering the tooth neck (cervix) at the lingual side or palatal side is pulled by the other one of them via the occlusal surface, which is a coupling portion, when about to be displaced under the pressure of the teeth before straightening, tensile force in a direction opposite to the direction of the displacement is produced. This tensile force is used as the orthodontic force. On the other hand, when an incisal or biting edge of a tooth has large holes decreasing the coupling portion, there is little action of pulling in an opposite direction to displacement, making it difficult for the tensile force to be produced. Thus, it becomes difficult for orthodontic force to be exerted. In the first embodiment, the mesh structure provides the coupling portion in the occlusal surface. Thereby, the orthodontic force is compensated, and the occlusal surface can be exposed.

When a general dental intraoral device is used, the occlusal surface is covered, which may cause side effects such as dental intrusion or recession of periodontal tissue, a change in the occlusal vertical dimension, and the associated sensation of the poor condition in the entire body. The dental intraoral device (orthodontic aligner 20) according to the first embodiment can reduce the area of contact with the teeth of the patient and a load thereon. Accordingly, the above symptoms can be reduced. Further, the dental intraoral device (orthodontic aligner 20) is relatively lighter. Thereby, the feeling of the foreign object for the patient can be reduced, and the falling off of the dental intraoral device due to its weight can be inhibited.

Moreover, the dental intraoral device (orthodontic aligner 20) according to the first embodiment can allow air to flow into the space between the dental intraoral device (orthodontic aligner 20) and the teeth 10 when the dental intraoral device (orthodontic aligner 20) is removed. This can make it easier to remove the dental intraoral device (orthodontic aligner 20).

Furthermore, the dental intraoral device (orthodontic aligner 20) according to the first embodiment improves the passability of saliva and is therefore effective in inhibiting intraoral drying, preventing ulcers and tooth decay resulting from drying, and accelerating wound healing. Moreover, forming the dental intraoral device into the mesh shape distributes stress on the dental intraoral device, and makes the dental intraoral device flexible and difficult to break.

In the dental intraoral device (orthodontic aligner 20) according to the first embodiment, at least a part of the tooth portion (anterior tooth portion 21, molar tooth portion 22) is formed by approximately regularly arranging the unit regions 31 having the holes 31b (FIGS. 4 to 6).

This can isotropically distribute force on the tooth portion (anterior tooth portion 21, molar tooth portion 22), and prevent the concentration of stress. Thus, plastic deformation and breakage can be avoided.

In the dental intraoral device (orthodontic aligner 20) according to the first embodiment, the cross-sectional shape of the unit region 31 is formed by the regular polygon that can tessellate (realize the tessellation) with one type of the polygon (FIGS. 4 to 6).

Thus, the tooth portion (anterior tooth portion 21, molar tooth portion 22) is provided with the unit regions 31 having uniform shapes without gaps therebetween. This can expose the occlusal surface and improve hygiene while ensuring a predetermined strength.

In the dental intraoral device (orthodontic aligner 20) according to the first embodiment, the length of each side of the unit region 31 is 0.1 mm or more (FIGS. 4 to 6).

This can make it easier for a resin to flow out from the holes 31b forming the unit regions 31, and avoid resin retention or the like when the dental intraoral device (orthodontic aligner 20) is manufactured by the three-dimensional modeling device.

### Second Embodiment

A dental intraoral device according to a second embodiment differs from the dental intraoral device according to the first embodiment in that the configuration of a tooth portion is different from that of the tooth portion in the first embodiment.

(Configuration of Dental Intraoral Device) FIG. 7 is a perspective view illustrating unit regions of a tooth portion according to the second embodiment. The configuration of the dental intraoral device according to the second embodiment is described below. Note that parts that are the same as or equivalent to those that have been described in the first embodiment are described by using the same terms and the same reference signs.

As illustrated in FIG. 7, a tooth portion (anterior tooth portion 21, molar tooth portion 22) according to the second embodiment is formed into a mesh shape by approximately regularly arranging unit regions 131 having holes 131b. Each of the unit regions 131 is formed by a regular hexahedron. The regular hexahedron is a polyhedron that can realize space-filling (fill a space) with one type of the polyhedron.

In the anterior tooth portion 21, the unit regions 131 are formed in a space having a predetermined height (thickness) H along a tip 11a, a buccal-side (facial-side) surface 11b, and a palatal-side surface 11c of an anterior tooth 11 by the regular hexahedron that can realize the space-filling (fill the space). Moreover, in a molar tooth portion 22, the unit regions 131 are formed in a space having the predetermined height H along an occlusal surface 12a, a buccal-side (facial-side) surface 12b, and a palatal-side surface 12c of a molar tooth 12 by a regular hexahedron that can realize the space-filling.

Each of the unit regions 131 is formed into a frame shape by a frame 131c defines the sides of the hexahedron and includes openings or holes 131b formed through the surfaces.

The anterior tooth portion 21 is formed by the unit regions 131 that can realize the space-filling. The unit regions 131 fill the space having the predetermined height H along the tip 11a, the buccal-side surface 11b, and the palatal-side surface 11c of the anterior tooth 11 as illustrated in FIG. 2, thereby forming the anterior tooth portion 21. The molar tooth portion 22 is formed by the unit regions 131 that can realize the space-filling. The unit regions 131 fill the space having the predetermined height H along the occlusal surface 12a, the buccal-side surface 12b, and the palatal-side surface 12c of the molar tooth 12, as illustrated in FIG. 3, thereby forming the molar tooth portion 22.

As illustrated in FIG. 7, a length L of each side of the unit region 131 may be, for example, 0.1 [mm] or more and 5.0 [mm] or less. A width W of the frame 131c may be set to a predetermined value (e.g., 0.02 [mm]). A ratio L/W of the width W to the length L is preferably 2.0 to 80, more preferably 2.5 to 70, and yet more preferably 3.0 to 60. The saliva easily passes when the ratio L/W of the width W to the length L is 2.0 or more. The frame 131c that defines the sides becomes difficult to buckle when the ratio L/W of the width W to the length L is 80 or less.

Note that the shape of the unit region 131 is not limited to the regular tetrahedron, and may be a polyhedron that can realize the space-filling (fill the space) with one type of the polyhedron, such as a truncated octahedron or a rhombic dodecahedron.

Moreover, the shape of the frame that defines the sides of the unit region 131 is not particularly limited but is preferably symmetrical with respect to the long axis of the frame. Specifically, the shape of the frame that defines the sides of the unit region 131 includes a circular column, a regular triangular prism, a regular quadratic prism, a regular pentangular prism, a regular hexagonal prism, any other regular prism, a circular cone, a regular triangular pyramid (regular tetrahedron), a square pyramid, any other regular pyramid, and the like. Among these, the shape of the frame that defines the sides of the unit region 131 is preferably the circular column, the regular triangular prism, the regular quadratic prism, the regular pentangular prism, the regular hexagonal prism, any other regular prism, and more preferably the circular column, the regular triangular prism, the regular quadratic prism, or the regular hexagonal prism.

(Effect of Dental Intraoral Device) The effect of the dental intraoral device (orthodontic aligner 20) according to the second embodiment is described below. **In** the dental intraoral device (orthodontic aligner 20) according to the second embodiment, each of the unit regions 131 is formed by the polyhedron that can realize the space-filling (fill the space) with one type of the polyhedron (FIG. 7).

Thus, the tooth portion (anterior tooth portion 21, molar tooth portion 22) can be provided with the unit regions 131 having uniform shapes without gaps. This can expose the occlusal surface and improve hygiene while ensuring a predetermined strength.

Note that other configurations and advantageous effects are approximately similar to those in the first embodiment described above, and the descriptions thereof are omitted.

### Third Embodiment

A dental intraoral device according to a third embodiment differs from the dental intraoral device according to the first embodiment in that the configuration of a tooth portion is different from that of the tooth portion in the first embodiment.

(Configuration of Dental Intraoral Device) FIG. 8 is an exploded perspective view illustrating an orthodontic aligner according to the third embodiment and an upper jaw. FIG. 9 is a perspective view illustrating a tooth portion covering a tooth to be straightened (straightened tooth 10) according to the third embodiment. The configuration of the dental intraoral device according to the third embodiment is described below. Note that parts that are the same as or equivalent to ones that have been described in the first and second embodiments are described by using the same terms or the same reference signs.

As illustrated in FIGS. 8 and 9, a molar tooth portion 22A covering the molar tooth 12A to be straightened is formed into a mesh shape by approximately regularly arranging the unit regions 31 having holes 31b. The orthodontic aligner 20 used for teeth other than the molar tooth portion 22A may be formed to be solid.

As illustrated in FIG. 9, a side portion (buccal-side portion 22b, palatal-side portion 22c) of the molar tooth portion 22A is formed with unit regions having higher rigidity than the occlusal portion 22a of the molar tooth portion 22A. In other words, the unit regions 31A of the side portion (buccal-side portion 22b, palatal-side portion 22c) of the molar tooth portion 22A are formed with the higher rigidity than the unit regions 31B of the top portion (occlusal portion 22a) of the molar tooth portion 22A. Note that FIG. 9 illustrates parts of the unit regions 31A and 31B in an enlarged view.

For example, the unit regions 31A of the side portion (buccal-side portion 22b, palatal-side portion 22c) of the molar tooth portion 22A can have the higher rigidity than the unit regions 31B of the top portion (occlusal portion 22a) of the molar tooth portion 22A by forming the unit regions 31A with smaller shapes than the unit regions 31B. More specifically, for example, the length of each side of the unit region 31A of the side portion (buccal-side portion 22b, palatal-side portion 22c) of the molar tooth portion 22A may be 0.1 [mm], and the length of each side of the unit region 31B of the top portion (occlusal portion 22a) of the molar tooth portion 22A may be 0.5 [mm].

Also, the thickness of each side wall of the unit region 31A of the side portion (buccal-side portion 22b, palatal-side portion 22c) of the molar tooth portion 22A is made thicker than the thickness of each side wall of the unit region 31B of the top portion (occlusal portion 22a) of the molar tooth portion 22A so that the rigidity of the unit region 31A can be higher than the rigidity of the unit region 31B.

(Effect of Dental Intraoral Device) The effect of the dental intraoral device (orthodontic aligner 20) according to the third embodiment is described below. In the dental intraoral device (orthodontic aligner 20) according to the third embodiment, the side portion (buccal-side portion 22b, palatal-side portion 22c) of the tooth portion (molar tooth portion 22A) covering the straightened tooth (molar tooth 12A) is formed with the unit regions having the higher rigidity than the top portion (occlusal portion 22a) of the tooth portion (molar tooth portion 22A) (FIG. 9).

For example, by forming the unit regions 31A of the side portion (buccal-side portion 22b, palatal-side portion 22c) of the tooth portion (molar tooth portion 22A) with a smaller shape than the unit regions 31B of the top portion (occlusal portion 22a) of the tooth portion (molar tooth portion 22A), the strength of the side portion (buccal-side portion 22b, palatal-side portion 22c) of the tooth portion (molar tooth portion 22A) can be higher than that of the top portion (occlusal portion 22a) of the tooth portion (molar tooth portion 22A).

Moreover, for example, by forming the unit regions 31A of the side portion (buccal-side portion 22b, palatal-side portion 22c) of the tooth portion (molar tooth portion 22A) in a denser shape than the unit regions 31B of the top portion (occlusal portion 22a) of the tooth portion (molar tooth portion 22A), the strength of the side portion (buccal-side portion 22b, palatal-side portion 22c) of the tooth portion (molar tooth portion 22A) can be higher than that of the top portion (occlusal portion 22a) of the tooth portion (molar tooth portion 22A).

Thus, when a tooth (molar tooth 12A) is straightened by the dental intraoral device (orthodontic aligner 20), the strong orthodontic force can be applied from the side portion (buccal-side portion 22b, palatal-side portion 22c) of the tooth portion (molar tooth portion 22A). As a result, the orthodontic force of the dental intraoral device (orthodontic aligner 20) on the tooth can be improved.

Note that other configurations and advantageous effects are approximately similar to those in the first and second embodiments described above, and accordingly, the description thereof is omitted.

The dental intraoral device according to the present disclosure has been described above in accordance with the first to third embodiments. However, the specific configurations are not limited to the ones of these embodiments, and design changes, additions, combinations of the embodiments, and the like are allowed without departing from the scope of the present invention defined by the appended claims.

In the first to third embodiments, as an example of the three-dimensional modeling device, the stereolithography apparatus using the photo-curing resin that is cured by ultraviolet laser light has been shown. However, the three-dimensional modeling device may be a projection type that cures and laminates a photo-curing resin by utilizing the light of a projector, may be an inkjet type that cures and laminates a liquid photo-curing resin by jetting the resin and applying ultraviolet light thereto, may be a fused deposition modeling type that piles up a thermoplastic resin layer by layer, or may be a powder sintering type that applies high-output laser light to a powder material and sinters the material.

In the first and third embodiments, the tooth portion (anterior tooth portion 21, molar tooth portion 22) is formed by one layer or stage of the unit regions. However, the tooth portion (anterior tooth portion 21, molar tooth portion 22) may be formed by two layers or stages of the unit regions as illustrated in FIG. 10 or may be formed by three or more layers or stages of the unit regions.

**In** the first and third embodiments, the cross-sectional shape of the unit region 31 is the regular polygon that can tessellate (realize the tessellation) with one type of the polygon (polygonal shape). However, the cross-sectional shape of the unit region may be a polygon that can tessellate with one type of the polygon (polygonal shape).

For example, the polygon that can tessellate (realize the tessellation) with one type of the polygon may be a parallelogram as illustrated in FIG. 11A. Alternatively, the polygon that can tessellate with one type of the polygon may be a parallelogram that is formed by combining two congruent triangles as illustrated in FIG. 11B. Alternatively, the polygon that can tessellate with one type of the polygon may be a parallel hexagon as illustrated in FIG. 12A. Alternatively, the polygon that can tessellate with one type of the polygon may be a parallel hexagon formed by combining two congruent quadrangles as illustrated in FIG. 12B. Alternatively, the polygon that can tessellate with one type of the polygon may be a parallel hexagon formed by combining two congruent pentagons as illustrated in FIG. 13A. Alternatively, the polygon that can tessellate with one type of the polygon may be a pentagon that can tessellate as illustrated in FIG. 13B.

Moreover, a polygon that can tessellate (realize the tessellation) with two or more types of the polygon may be a regular polygon that is an Archimedes' tessellation shape. For example, the polygon that can tessellate with two or more types of the polygon may be a shape formed by eight regular triangles and one regular hexagon, as illustrated in FIG. 14.

A polygon that can tessellate preferably a combination of one or more of a triangle, a quadrangle, a pentagon, a regular polygon, and a parallel hexagon. The polygon that can tessellate may be more preferably a combination of one or more of the regular polygon, the parallelogram, and the parallel hexagon. The polygon that can tessellate may be yet more preferably a combination of one or more of the regular triangle, the regular tetragon, and the regular hexagon. The improvement of the symmetry of the unit region makes it difficult for a given load to concentrate and makes it difficult for plastic deformation and breakage to be caused. Particularly, in the regular triangle, the regular tetragon, and the regular hexagon, a given load is isotropically distributed, and thus plastic deformation and breakage are significantly inhibited.

In other words, the cross-sectional shape of the unit region may be formed by a regular polygon that can tessellate (realize the tessellation), may be formed by a polygon that can tessellate, or may be formed by a figure that can tessellate.

In the second embodiment, the unit region 131 is the regular hexagon (Archimedes' regular quadratic prism) that can realize the space-filling (fill the space) with one type of the polyhedron. However, the unit region may be a uniform polyhedron that can realize the space-filling with one type of the polyhedron.

The uniform polyhedron that can realize the space-filling with one type of the polyhedron may be, for example, an Archimedes' regular triangular prism, Archimedes' regular hexagonal prism, a truncated octahedron, a rhombic dodecahedron, or the like.

The unit region may be a polyhedron that can realize the space-filling (fill the space) with one type of the polyhedron. The polyhedron that can realize the space-filling with one type of the polyhedron may be, for example, a gyrobifastigium (Johnson solid J26) or the like.

The unit region may be a uniform polyhedron that can realize the space-filling (fill the space) by two or more types of the polyhedron. The uniform polyhedron that can realize the space-filling by two or more types of the polyhedron may be, for example, a uniform polyhedron that consists of a regular tetrahedron and a regular octahedron, a uniform polyhedron that consists of a regular tetrahedron and a truncated tetrahedron, a uniform polyhedron that consists of a regular octahedron and a truncated hexahedron, a uniform polyhedron that consists of a regular octahedron and a cuboctahedron, or a uniform polyhedron that consists of a rhombitruncated cuboctahedron and a regular octagonal prism.

Alternatively, the uniform polyhedron that can realize the space-filling (fill the space) by two or more types of the polyhedron may be, for example, a uniform polyhedron that consists of a truncated tetrahedron, a truncated octahedron, and a cuboctahedron, a uniform polyhedron that consists of a truncated tetrahedron, a truncated hexahedron, and a rhombitruncated cuboctahedron, a uniform polyhedron that consists of a regular tetrahedron, a cube, and a rhombicuboctahedron, a uniform polyhedron that consists of a cube, a cuboctahedron, and a rhombicuboctahedron, or a uniform polyhedron that consists of a cube, a truncated octahedron, and a rhombitruncated cuboctahedron.

Alternatively, a uniform polyhedron that can realize the space-filling (fill the space) by two or more types of the polyhedron may be, for example, a combination of a cube, a truncated hexahedron, a rhombitruncated cuboctahedron, and a regular octagonal prism, or a combination of various types of equilateral rhombic polyhedrons.

The unit region may be a polyhedron that can realize the space-filling (fill the space) by two or more types of the polyhedron. The polyhedron that can realize the space-filling by two or more types of the polyhedron may be, for example, a polyhedron that consists of Johnson solid J1 (square pyramid) and Johnson solid J3 (triangular cupola), a polyhedron that consists of Johnson solid J1 (square pyramid) and Johnson solid J7 (elongated triangular pyramid), a polyhedron that consists of Johnson solid J1 and Johnson solid J27 (triangular orthobicupola), a polyhedron that consists of a regular tetrahedron and Johnson solid J1, a polyhedron that consists of a regular tetrahedron and Johnson solid J4 (square cupola), a polyhedron that consists of a regular tetrahedron and Johnson solid J8 (elongated square pyramid), a polyhedron that consists of a regular tetrahedron and Johnson solid J28 (square orthobicupola), a polyhedron that consists of a regular octahedron and Johnson solid J3, a polyhedron that consists of a regular octahedron and Johnson solid J7 (elongated triangular pyramid), a polyhedron that consists of a regular octahedron and Johnson solid J12 (triangular bipyramid), a polyhedron that consists of a truncated tetrahedron and Johnson solid J12, a polyhedron that consists of a truncated hexahedron and Johnson solid J1, or a polyhedron that consists of a cuboctahedron and Johnson solid J1.

Moreover, the polyhedron that can realize the space-filling (fill the space) by two or more types of the polyhedron may be, for example, a polyhedron that consists of a regular tetrahedron, Johnson solid J1, and Johnson solid J18 (elongated triangular cupola), a polyhedron that consists of a regular tetrahedron, Johnson solid J1, and Johnson solid J35 (elongated triangular orthobicupola), a polyhedron that consists of a regular tetrahedron, Johnson solid J1, and Johnson solid J36 (elongated triangular gyrobicupola), a polyhedron that consists of a regular tetrahedron, Johnson solid J1, and Johnson solid J15 (elongated square bipyramid), a polyhedron that consists of a regular tetrahedron, a regular hexahedron, and Johnson solid J28, a polyhedron that consists of a regular tetrahedron, a regular octahedron, and Johnson solid J15, a polyhedron that consists of a regular hexahedron, a regular dodecahedron, and Johnson solid J91 (bilunabirotunda), a polyhedron that consists of a regular hexahedron, a cuboctahedron, and Johnson solid J4, a polyhedron that consists of a regular hexahedron, a cuboctahedron, and Johnson solid J19 (elongated square cupola), a polyhedron that consists of a regular hexahedron, a cuboctahedron, and Johnson solid J28, a polyhedron that consists of a regular hexahedron, a regular tetrahedron, and Johnson solid J19, a polyhedron that consists of a regular octahedron, Johnson solid J1, and Johnson solid J3, or a polyhedron that consists of a regular octahedron, Johnson solid J1, and Johnson solid J7.

Moreover, the polyhedron that can realize the space-filling (fill the space) by two or more types of the polyhedron may be, for example, a polyhedron that consists of one or a combination of a regular tetrahedron, a regular hexahedron, and a cuboctahedron [Johnson solid J28 and Johnson solid J29 (square gyrobicupola)], a polyhedron that consists of one or a combination of a regular tetrahedron and Johnson solid J1 [a regular hexahedron, Johnson solid J8, and Johnson solid J15] and one or a combination of [Johnson solid J28 and Johnson solid J29], a polyhedron that consists of a regular tetrahedron, a regular hexahedron, a cuboctahedron, and Johnson solid J37 (elongated square gyrobicupola), a polyhedron that consists of a regular tetrahedron, a regular hexahedron, Johnson solid J1, and Johnson solid J8, a polyhedron that consists of a regular tetrahedron, a regular octahedron, Johnson solid J1, and Johnson solid J15, a polyhedron that consists of a regular tetrahedron, Johnson solid J8, Johnson solid J15, and Johnson solid J19, a polyhedron that consists of one or a combination of a regular tetrahedron, Johnson solid J1, and Johnson solid J28 [a regular hexahedron, Johnson solid J8, and Johnson solid J15], a polyhedron that consists of one or a combination of a regular tetrahedron, Johnson solid J1, and Johnson solid J37 [a regular hexahedron, Johnson solid J8, and Johnson solid J15], a polyhedron that consists of one or a combination of a regular tetrahedron, a regular hexahedron, Johnson solid J1, and Johnson solid J19 [Johnson solid J8 and Johnson solid J15], or a polyhedron that consists of one or a combination of a regular tetrahedron, Johnson solid J1, and Johnson solid J4 [a regular hexahedron, Johnson solid J8 and Johnson solid J15].

The polyhedron that can realize the space-filling (fill the space) may be preferably a combination of one or more types of a regular polyhedron, a semi-regular polyhedron, a regular prism, a regular antiprism, and a Johnson solid. The polyhedron that can realize the space-filling may be more preferably a combination of one or more types of equilateral rhombic polyhedrons such as a regular polyhedron, a cuboctahedron, a regular polygonal prism, and a rhombic dodecahedron, a parallelepiped, a truncated octahedron, a parallelohedron such as an elongated rhombic dodecahedron, a rhombicuboctahedron, and a rhombitruncated cuboctahedron. The polyhedron that can realize the space-filling may be yet more preferably a combination of one or more types of a regular tetrahedron, a regular hexahedron (cube), a regular octahedron, a regular triangular prism, a regular quadrangular prism (cuboid), a regular hexagonal prism, a truncated octahedron, a rhombic dodecahedron, and an elongated rhombic dodecahedron.

The improvement of the symmetry of the unit region makes it difficult for a given load to concentrate and makes it difficult for plastic deformation and breakage to be caused. Particularly, in the regular tetrahedron, a regular hexahedron (cube), a regular octahedron, a regular triangular prism, a regular quadrangular prism (cuboid), a regular hexagonal prism, a truncated octahedron, a rhombic dodecahedron, and an elongated rhombic dodecahedron, a given load is isotropically distributed, and thus plastic deformation and breakage are significantly inhibited.

In other words, the unit region may be formed by a uniform polyhedron that can realize the space-filling (fill the space), may be formed by a polyhedron that can realize the space-filling, or may be formed by a solid that can realize the space-filling.

In the first and second embodiments, the tooth portion (anterior tooth portion 21, molar tooth portion 22) is entirely formed into a mesh shape by approximately regularly arranging the unit regions 31, 131 having the holes 31b, 131b. However, the tooth portion may be partially formed into a mesh shape by approximately regularly arranging the unit regions having the holes. For example, by forming only the molar tooth portion into a mesh shape by approximately regularly arranging the unit regions having the holes, the molar tooth portion that is subjected to more force than the anterior tooth portion when biting may be more rigid than the anterior tooth portion. The shape of the hole is not particularly limited but is preferably highly symmetrical. Specifically, the cross-sectional shape of the hole may be a circle, a regular triangle, a regular tetragon, a regular pentagon, a regular hexagon, or any other regular polygon. The cross-sectional shape of the hole is more preferably a circle, a regular triangle, a regular tetragon, or a regular hexagon. The cross-sectional shape of the hole is yet more preferably a circle since the circle has no notch that may be an origin of breaking is formed.

In the first to third embodiments, the sides that define each unit region are straight lines. However, the sides that define each unit region may be curved lines as long as the effects of the present disclosure are not maintained. For example, a curvature radius R is preferably equal to or more than 0.3 times the length L of the side of the unit region, more preferably equal to or more than 0.5 times, and yet more preferably equal to or more than 1.0 times. The upper limit of the curvature radius R is not particularly limited but may be, for example, equal to or less than 100 times the length L of the side of the unit region.

In the first to third embodiments, the side walls 31a and 131a are solid. However, a part of the side wall may have a hole. Although not particularly limited, the shape of the hole is preferably highly symmetrical. Specifically, the cross-sectional shape of the hole may be a circle, a regular triangle, a regular tetragon, a regular pentagon, a regular hexagon, or any other regular polygon. The cross-sectional shape of the hole is more preferably a circle, a regular triangle, a regular tetragon, or a regular hexagon. The cross-sectional shape of the hole is yet more preferably a circle since the circle has no notch that may be an origin of breaking is formed.

In the first and third embodiments, a reinforcing structure is not provided between side walls. However, as illustrated in FIG. 15, a reinforcing structure such as braces 35 may be provided between the side walls 31a.

In the third embodiment, the buccal-side portion 22b and the palatal-side portion 22c of the molar tooth portion 22A to be straightened are formed with the unit regions having higher rigidity than the occlusal portion 22a of the molar tooth portion 22A. However, the buccal-side portion and the palatal-side portion of the molar tooth portion to be straightened may be formed with unit regions having the same rigidity as the occlusal portion of the molar tooth portion.

In the third embodiment, the tooth 10 to be straightened is the molar tooth 12A. However, the tooth to be straightened may be the anterior tooth.

In the third embodiment, the orthodontic aligner 20 used for other than the straightened molar tooth portion 22A is formed to be solid. However, the orthodontic aligner 20 used for other than the molar tooth portion 22A may be formed into a mesh shape. Moreover, the orthodontic aligner may be locally formed with the unit regions having higher rigidity only for a region where strong force particularly needs to be applied or a region where durability against strong force is particularly required. For example, only a region covering the tooth neck (cervix) of a tooth that needs to be moved may be formed with the unit regions having higher rigidity, only a region near the tooth root among teeth that need to be moved may be formed with the unit regions having higher rigidity, or only the buccal-side (facial-side) portion, the palatal-side portion of the anterior tooth portion, and the occlusal portion where stress tends to concentrate during attachment and detachment may be formed with the unit regions having higher rigidity.

Moreover, the anterior tooth part of the tooth portion may be formed with the unit regions having higher rigidity than other parts of the tooth portion. In this case, the strength of the anterior tooth part that is subjected to high stress during the attachment and detachment of the dental intraoral device may be heightened.

Moreover, the side portion of the tooth portion may be formed with the unit regions having higher rigidity than the top portion of the tooth portion. In this case, contact with the teeth is minimized in the top portion of the tooth portion, and the side portion of the tooth portion has a thickness required for the dental intraoral device. Thus, intrusion of the teeth resulting as a side effect from the covering of the top portion of the tooth portion is inhibited, nevertheless, the function of the dental intraoral device may be exhibited. For example, orthodontic force is exhibited in the case of the orthodontic aligner, performance of retaining teeth is exhibited in the case of an orthodontic retainer, and impact absorbing performance is exhibited in the case of a mouth guard for sports. Moreover, the anterior tooth part of the tooth portion may be formed with the unit regions having higher rigidity than other parts of the tooth portion, and the side portion of the tooth portion may be formed with the unit regions having higher rigidity than the top portion of the tooth portion.

In the first to third embodiments, the dental intraoral device according to the present disclosure is applied to the orthodontic aligner 20 to be worn within the oral cavity to cover the upper-jaw teeth 10. However, the dental intraoral device according to the present disclosure may also be applied to an orthodontic aligner to be worn within the oral cavity to cover lower-jaw teeth.

In the first to third embodiments, the dental intraoral device according to the present disclosure is applied to the orthodontic aligner 20 that does not have a base or bed portion. However, the dental intraoral device according to the present disclosure may also be applied to an orthodontic aligner that has a base or bed portion.

In the first to third embodiments, the dental intraoral device according to the present disclosure is applied to the orthodontic aligner.

## Claims

1. An orthodontic aligner (20) that is worn within an oral cavity, comprising:
resin tooth portions (21, 22) which comprise:
an anterior tooth portion (21) for covering anterior teeth (11); and
a molar tooth portion (22) for covering molar teeth (12),
**characterised in that**
the anterior tooth portion (21) and the molar tooth portion (22) are each formed into a mesh shape by approximately regularly arranging unit regions (31) each of which comprises a hole (31b),
wherein in a top portion of the tooth portions, the hole (31b) extends in a vertical direction, and in side portions of the tooth portions, the hole (31b) extends in a horizontal direction,
wherein a length (L) of each side of the unit region (31) is from 0.1 mm or more to 5.0 mm or less.

2. The orthodontic aligner (20) according to claim 1,
wherein a cross-sectional shape of each of the unit regions (31) is formed by a figure that can realize a tessellation, preferably a polygon that can realize a tessellation, more preferably a regular polygon that can realize a tessellation.

3. The orthodontic aligner (20) according to claim 1 or claim 2,
wherein a cross-sectional shape of each of the unit regions (31) is formed by a polygon that can realize a tessellation with one type of the polygon preferably a regular polygon that can realize a tessellation with one type of the polygon.

4. The orthodontic aligner (20) according to claim 1,
wherein the unit region (131) is formed by a solid that can realize space-filling.

5. The orthodontic aligner (20) according to claim 1 or 4,
wherein the unit region (131) is formed by a polyhedron that can realize space-filling.

6. The orthodontic aligner (20) according to any one of claims 1, 4, and 5,
wherein the unit region (131) is formed by a uniform polyhedron that can realize space-filling.

7. The orthodontic aligner (20) according to any one of claims 1 and 4 to 6,
wherein the unit region (131) is formed by a polyhedron that can realize space-filling with one type of the polyhedron.

8. The orthodontic aligner (20) according to any one of claims 1 and 4 to 7,
wherein the unit region (131) is formed by a uniform polyhedron that can realize space-filling with one type of the polyhedron.

9. The orthodontic aligner (20) according to any one of claims 1 to 8,
wherein an anterior tooth part of the tooth portion (21) is formed by the unit regions (31, 131) having higher rigidity than other parts of the tooth portion (21).

10. The orthodontic aligner (20) according to any one of claims 1 to 9,
wherein the side portions (22b, 22c) of the tooth portion (22A) are formed by the unit regions (31A) having higher rigidity than the top portion (22a) of the tooth portion (22A).

11. The orthodontic aligner (20) according to any one of claims 1 to 10,
wherein the tooth portion (21, 22, 22A) is formed to cover intraoral teeth (10, 11, 12, 12A), and
a part of the tooth portion (21, 22, 22A) where the unit regions (31, 31A, 31B, 131) are formed is the tooth portion (21, 22, 22A) that covers a tooth (10, 11, 12, 12A) to be straightened.

12. The orthodontic aligner (20) according to claim 11,
wherein the side portions (22b, 22c) of the tooth portion (22A) that covers the tooth (10, 12, 12A) to be straightened are formed by the unit regions (31A) having higher rigidity than the top portion (22a) of the tooth portion (22A).

13. A method for manufacturing the orthodontic aligner (20) according to any one of claims 1 to 12, the method comprising:
generating three-dimensional data of the orthodontic aligner (20); and
modeling the orthodontic aligner (20) by a three-dimensional modeling device based on the three-dimensional data.

## Patentansprüche

1. Kieferorthopädische Aligner-Schiene (20), die in einer Mundhöhle getragen wird, umfassend:
aus Harz bestehende Zahnabschnitte (21, 22), die Folgendes umfassen:
einen Frontzahnabschnitt (21) zum Abdecken von Frontzähnen (11) und
einen Backenzahnabschnitt (22) zum Abdecken von Backenzähnen (12), **dadurch gekennzeichnet, dass** der Frontzahnabschnitt (21) und der Backenzahnabschnitt (22) jeweils dadurch zu einer Gitterform ausgebildet sind, dass die Einheitenbereiche (31), die jeweils ein Loch (31b) umfassen, annähernd regelmäßig angeordnet sind,
wobei sich in einem oberen Abschnitt der Zahnabschnitte das Loch (31b) in vertikaler Richtung erstreckt und sich in Seitenabschnitten der Zahnabschnitte das Loch (31b) in horizontaler Richtung erstreckt,
wobei die Länge (L) jeder Seite des Einheitenbereichs (31) 0,1 mm oder mehr bis 5,0 mm oder weniger beträgt.

2. Kieferorthopädische Aligner-Schiene (20) nach Anspruch 1,
wobei eine Querschnittsform jedes Einheitenbereichs (31) durch eine Figur ausgebildet ist, mit der eine Parkettierung umgesetzt werden kann, vorzugsweise durch ein Vieleck, mit dem eine Parkettierung umgesetzt werden kann, insbesondere durch ein reguläres Vieleck, mit dem eine Parkettierung umgesetzt werden kann.

3. Kieferorthopädische Aligner-Schiene (20) nach Anspruch 1 oder Anspruch 2,
wobei eine Querschnittsform jedes Einheitenbereichs (31) durch ein Vieleck ausgebildet ist, mit dem eine Parkettierung mit einer einzigen Sorte des Vielecks umgesetzt werden kann, vorzugsweise durch ein reguläres Vieleck, mit dem eine Parkettierung mit einer einzigen Sorte des Vielecks umgesetzt werden kann.

4. Kieferorthopädische Aligner-Schiene (20) nach Anspruch 1,
wobei der Einheitenbereich (131) durch einen Körper ausgebildet ist, mit dem eine Raumfüllung umgesetzt werden kann.

5. Kieferorthopädische Aligner-Schiene (20) nach Anspruch 1 oder 4,
wobei der Einheitenbereich (131) durch ein Polyeder ausgebildet ist, mit dem eine Raumfüllung umgesetzt werden kann.

6. Kieferorthopädische Aligner-Schiene (20) nach einem der Ansprüche 1, 4 und 5,
wobei der Einheitenbereich (131) durch ein gleichmäßiges Polyeder ausgebildet ist, mit dem eine Raumfüllung umgesetzt werden kann.

7. Kieferorthopädische Aligner-Schiene (20) nach einem der Ansprüche 1 und 4 bis 6,
wobei der Einheitenbereich (131) durch ein Polyeder ausgebildet ist, mit dem eine Raumfüllung mit einer einzigen Sorte des Polyeders umgesetzt werden kann.

8. Kieferorthopädische Aligner-Schiene (20) nach einem der Ansprüche 1 und 4 bis 7,
wobei der Einheitenbereich (131) durch ein gleichmäßiges Polyeder ausgebildet ist, mit dem eine Raumfüllung mit einer einzigen Sorte des Polyeders umgesetzt werden kann.

9. Kieferorthopädische Aligner-Schiene (20) nach einem der Ansprüche 1 bis 8,
wobei ein Frontzahnteil des Zahnabschnitts (21) durch die Einheitenbereiche (31, 131) ausgebildet ist, die eine höhere Steifigkeit als andere Teile des Zahnabschnitts (21) aufweisen.

10. Kieferorthopädische Aligner-Schiene (20) nach einem der Ansprüche 1 bis 9,
wobei die Seitenabschnitte (22b, 22c) des Zahnabschnitts (22A) durch die Einheitenbereiche (31A) ausgebildet sind, die eine höhere Steifigkeit als der obere Abschnitt (22a) des Zahnabschnitts (22A) aufweisen.

11. Kieferorthopädische Aligner-Schiene (20) nach einem der Ansprüche 1 bis 10,
wobei der Zahnabschnitt (21, 22, 22A) so ausgebildet ist, dass er die intraoralen Zähne (10, 11, 12, 12A) abdeckt, und
es sich bei einem Teil des Zahnabschnitts (21, 22, 22A), an dem die Einheitenbereiche (31, 31A, 31B, 131) ausgebildet sind, um den Zahnabschnitt (21, 22, 22A) handelt, der einen Zahn (10, 11, 12, 12A) abdeckt, der begradigt werden soll.

12. Kieferorthopädische Aligner-Schiene (20) nach Anspruch 11,
wobei die Seitenabschnitte (22b, 22c) des Zahnabschnitts (22A), der den Zahn (10, 12, 12A) abdeckt, der begradigt werden soll, durch die Einheitenbereiche (31A) ausgebildet sind, die eine höhere Steifigkeit als der obere Abschnitt (22a) des Zahnabschnitts (22A) aufweisen.

13. Verfahren zum Herstellen der kieferorthopädischen Aligner-Schiene (20) nach einem der Ansprüche 1 bis 12, wobei das Verfahren Folgendes umfasst:
Erzeugen von dreidimensionalen Daten der kieferorthopädischen Aligner-Schiene (20) und Modellieren der kieferorthopädischen Aligner-Schiene 20 durch eine dreidimensionale Modellierungsvorrichtung auf der Grundlage der dreidimensionalen Daten.

## Revendications

1. Gouttière orthodontique (20) qui est portée au sein d'une cavité buccale, comprenant :
des parties dents en résine (21, 22) qui comprennent :
une partie dents antérieures (21) pour recouvrir les dents antérieures (11) ; et
une partie molaires (22) pour recouvrir les molaires (12),
**caractérisée en ce que**
la partie dents antérieures (21) et la partie molaires (22) sont chacune en forme de treillis en disposant approximativement régulièrement des régions unitaires (31) dont chacune comprend un trou (31b),
dans laquelle, dans une partie supérieure des parties dents, l'orifice (31b) s'étend dans une direction verticale, et dans des parties latérales des parties dents, le trou (31b) s'étend dans une direction horizontale,
dans laquelle une longueur (1) de chaque côté de la région unitaire (31) est supérieure ou égale à 0,1 mm et inférieure ou égale à 5,0 mm.

2. Gouttière orthodontique (20) selon la revendication 1,
dans laquelle une forme de section transversale de chacune des régions unitaires (31) est formée par une figure qui peut réaliser une tessellation, de préférence un polygone qui peut réaliser une tessellation, plus préférablement un polygone régulier qui peut réaliser une tessellation.

3. Gouttière orthodontique (20) selon la revendication 1 ou la revendication 2,
dans laquelle une forme de section transversale de chacune des régions unitaires (31) est formée par un polygone qui peut réaliser une tessellation avec un type du polygone, de préférence un polygone régulier qui peut réaliser une tessellation avec un type du polygone.

4. Gouttière orthodontique (20) selon la revendication 1,
dans laquelle la région unitaire (131) est formée par un solide qui peut effectuer un comblement d'espace.

5. Gouttière orthodontique (20) selon la revendication 1 ou 4,
dans laquelle la région unitaire (131) est formée par un polyèdre qui peut effectuer un comblement d'espace.

6. Gouttière orthodontique (20) selon l'une quelconque des revendications 1, 4, et 5,
dans laquelle la région unitaire (131) est formée par un polyèdre uniforme qui peut réaliser un comblement d'espace.

7. Gouttière orthodontique (20) selon l'une quelconque des revendications 1 et 4 à 6,
dans laquelle la région unitaire (131) est formée par un polyèdre qui peut réaliser un comblement d'espace avec un type du polyèdre.

8. Gouttière orthodontique (20) selon l'une quelconque des revendications 1 et 4 à 7,
dans laquelle la région unitaire (131) est formée par un polyèdre uniforme qui peut réaliser un comblement d'espace avec un type du polyèdre.

9. Gouttière orthodontique (20) selon l'une quelconque des revendications 1 à 8,
dans laquelle une partie dents antérieures de la partie dents (21) est formée par les régions unitaires (31, 131) ayant une rigidité supérieure à celle d'autres parties de la partie dents (21).

10. Gouttière orthodontique (20) selon l'une quelconque des revendications 1 à 9,
dans laquelle les parties latérales (22b, 22c) de la partie dents (22A) sont formées par les régions unitaires (31A) présentant une rigidité supérieure à celle de la partie supérieure (22a) de la partie dents (22A).

11. Gouttière orthodontique (20) selon l'une quelconque des revendications 1 à 10,
dans laquelle la partie dents (21, 22, 22A) est formée pour recouvrir les dents intrabuccales (10, 11, 12, 12A), et
une partie de la partie dents (21, 22, 22A) où sont formées les régions unitaires (31, 31A, 31B, 131) est la partie dents (21, 22, 22A) qui recouvre une dent (10, 11, 12, 12A) à redresser.

12. Gouttière orthodontique (20) selon la revendication 11,
dans laquelle les parties latérales (22b, 22c) de la partie dents (22A) qui recouvre la dent (10, 12, 12A) à redresser sont formées par les régions unitaires (31A) présentant une rigidité supérieure à celle de la partie supérieure (22a) de la partie de dents (22A).

13. Procédé de fabrication de la gouttière orthodontique (20) selon l'une quelconque des revendications 1 à 12, le procédé comprenant :
la génération de données tridimensionnelles de la gouttière orthodontique (20) ; et
la modélisation de la gouttière orthodontique (20) par un dispositif de modélisation tridimensionnelle sur la base des données tridimensionnelles.
